# EUROPEAN PATENT APPLICATION

(11) **EP 0 969 015 A2**
(43) Date of publication of application: **05.01.2000**
(21) Application number: 99304636.6
(22) Date of filing: 15.06.1999
(51) Int. Cl.: C07K 14/47, C07K 14/415, C07K 5/062, C07K 5/083, C07K 5/087, C07K 5/103, C07K 5/107, C07K 7/06, G01N 33/68, G01N 33/94

(54) **Peptide diagnostic markers for human disorders**

(30) Priority: 15.06.1998 US 89237 P; 15.06.1998 US 89238 P; 24.05.1999 US 317702 P
(71) Applicant: Ortho-Clinical Diagnostics, Inc., Rochester, NY 14626-5101 (US)
(72) Inventor: Shanahan, Michelle R., Palmyra, New York 14522 (US); Venturini, Albert J., East Stroudsburg, Pennsylvania 18301 (US); Daiss, John L., Rochester, New York 14613 (US); Friedman, Alan E., Rochester, New York 14617 (US)
(74) Representative: Mercer, Christopher Paul

(57) **Abstract**

The present invention broadly relates to human disorders including, but not limited to, metabolic and nutritional disorders, gastrointestinal disorders, central nervous system disorders, and autoimmunity or reduced immunity disorders.

The present invention more specifically relates to diagnostic markers comprising isolated peptides originally found in human tissue and body fluids and derivatives thereof. These peptides have been found to consistently exhibit opiate-like activity and/or are derivatives of peptides that exhibit opiate-like activity.

### Annex to the description

SEQUENCE LISTING

<110> Friedman, Alan
      Shanahan, Michelle
      Venturini, Al
      Daiss, Jack

<120> DIAGNOSTIC MARKERS FOR HUMAN DISORDERS

<130> CDS177 Jim Harrington

<140> 60/089,237
<141> 1998-05-15

<160> 43

<170> PatentIn Ver. 2.0

<210> 1
<211> 7
<212> PRT
<213> Protein

<400> 1
Tyr Pro Phe Pro Gly Pro Ile
  1               5


<210> 2
<211> 6
<212> PRT
<213> Protein

<400> 2
Tyr Pro Phe Pro Gly Pro
  1               5


<210> 3
<211> 5
<212> PRT
<213> Protein

<400> 3
Tyr Pro Phe Pro Gly
  1               5


<210> 4
<211> 4
<212> PRT
<213> Protein

<400> 4
Tyr Pro Phe Pro
1

<210> 5
<211> 3
<212> PRT
<213> Protein

<400> 5
Tyr Pro Phe
1

<210> 6
<211> 6
<212> PRT
<213> Protein

<400> 6
Pro Phe Pro Gly Pro Ile
1               5

<210> 7
<211> 8
<212> PRT
<213> Protein

<400> 7
Tyr Pro Gln Pro Gln Pro Phe Pro
1               5

<210> 8
<211> 6
<212> PRT
<213> Protein

<400> 8
Tyr Pro Gln Pro Gln Pro
1               5

<210> 9
<211> 5
<212> PRT
<213> Protein

<400> 9
Tyr Pro Gln Pro Gln
1               5

<210> 10
<211> 4
<212> PRT
<213> Protein

<400> 10
Tyr Pro Gln Pro
1

<210> 11
<211> 3
<212> PRT
<213> Protein

<400> 11
Tyr Pro Gln
1

<210> 12
<211> 6
<212> PRT
<213> Protein

<400> 12
Pro Gln Pro Gln Pro Phe
1               5

<210> 13
<211> 7
<212> PRT
<213> Protein

<400> 13
Tyr Ala Phe Gly Tyr Pro Ser
1               5

<210> 14
<211> 6
<212> PRT
<213> Protein

<400> 14
Tyr Ala Phe Gly Tyr Pro
1               5

<210> 15
<211> 5
<212> PRT
<213> Protein

<400> 15
Tyr Ala Phe Gly Tyr
1               5

<210> 16
<211> 4
<212> PRT
<213> Protein

<400> 16
Tyr Ala Phe Gly
1

<210> 17
<211> 3
<212> PRT
<213> Protein

<400> 17
Tyr Ala Phe
1

<210> 18
<211> 6
<212> PRT
<213> Protein

<400> 18
Ala Phe Gly Tyr Pro Ser
1               5

<210> 19
<211> 7
<212> PRT
<213> Protein

<400> 19
Tyr Ala Phe Glu Val Val Gly
1               5

<210> 20
<211> 6
<212> PRT
<213> Protein

<400> 20
Tyr Ala Phe Glu Val Val
1               5

<210> 21
<211> 5
<212> PRT
<213> Protein

<400> 21
Tyr Ala Phe Glu Val
1               5


<210> 22
<211> 4
<212> PRT
<213> Protein

<400> 22
Tyr Ala Phe Glu
1


<210> 23
<211> 6
<212> PRT
<213> Protein

<400> 23
Ala Phe Glu Val Val Gly
1               5


<210> 24
<211> 8
<212> PRT
<213> Protein

<400> 24
Phe Leu Phe Gln Pro Gln Arg Phe
1               5


<210> 25
<211> 7
<212> PRT
<213> Protein

<400> 25
Phe Leu Phe Gln Pro Gln Arg
1               5


<210> 26
<211> 6
<212> PRT
<213> Protein

<400> 26
Phe Leu Phe Gln Pro Gln
1               5

<210> 27
<211> 5
<212> PRT
<213> Protein

<400> 27
Phe Leu Phe Gln Pro
1               5

<210> 28
<211> 4
<212> PRT
<213> Protein

<400> 28
Phe Leu Phe Gln
1

<210> 29
<211> 3
<212> PRT
<213> Protein

<400> 29
Phe Leu Phe
1

<210> 30
<211> 7
<212> PRT
<213> Protein

<400> 30
Tyr Ala Phe Asp Val Val Gly
1               5

<210> 31
<211> 6
<212> PRT
<213> Protein

<400> 31
Tyr Ala Phe Asp Val Val
1               5

<210> 32
<211> 5
<212> PRT
<213> Protein

<400> 32
Tyr Ala Phe Asp Val
1               5

<210> 33
<211> 4
<212> PRT
<213> Protein

<400> 33
Tyr Ala Phe Asp
1

<210> 34
<211> 6
<212> PRT
<213> Protein

<400> 34
Ala Phe Asp Val Val Gly
1               5

<210> 35
<211> 6
<212> PRT
<213> Protein

<400> 35
Ala Pro Phe Xaa Gly Xaa
1               5

<210> 36
<211> 6
<212> PRT
<213> Protein

<400> 36
Ala Pro Phe Xaa Gly Xaa
1               5

<210> 37
<211> 5
<212> PRT
<213> Protein

<400> 37
Ala Pro Phe Xaa Gly
1               5

<210> 38
<211> 4
<212> PRT
<213> Protein

<400> 38
Ala Pro Phe Xaa
1

<210> 39
<211> 3
<212> PRT
<213> Protein

<400> 39
Ala Pro Phe
1

<210> 40
<211> 2
<212> PRT
<213> Protein

<400> 40
Ala Pro
1

<210> 41
<211> 5
<212> PRT
<213> Protein

<400> 41
Pro Phe Xaa Gly Xaa
1               5

<210> 42
<211> 5
<212> PRT
<213> Protein

<400> 42
Tyr Pro Phe Pro Pro
1               5

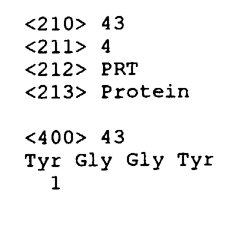

## Description

### Field of the Invention

The present invention broadly relates to human disorders including, but not limited to, metabolic and nutritional disorders, gastrointestinal disorders, central nervous system disorders, and autoimmunity or reduced immunity disorders.

The present invention more specifically relates to diagnostic markers comprising isolated peptides originally found in human tissue and body fluids and derivatives thereof. These peptides have been found to consistently exhibit opiate-like activity or are derivatives of peptides which exhibit opiate-like activity.

The present invention also relates to methods for diagnosis of the disorders by identification of these peptides as well as methods for detection of these peptides. Methods include but are not limited to Immunoassay tests and Mass Spectrometry.

In particular, the present invention relates to methods for diagnosing human disorders, such as a diet-related form of autism, by detection or quantitation of the peptides.

The present invention also relates to enzymes involved in catabolism of proline-rich peptides. An example of such an enzyme is dipeptidyl Peptidase IV.

The present invention also relates to pharmaceutical compositions for treatment of human disorders.

### Background of the Invention

The present invention is directed to "human disorders". For purposes of this application "human disorders" is defined to include "metabolic and nutritional disorders", "gastrointestinal disorders", "central nervous system disorders", "autoimmunity or reduced immunity disorders" and cancer.

For purposes of this application "metabolic and nutritional disorders" are defined as any condition or manifestation that involves "over absorption" or "under absorption" of a food stuff, vitamin, mineral or element, cofactor or salt, elements essential for health, or are involved in the interruption of digestion, a nutritional deficiency or toxicity. Further metabolic disorders may also include breakdown of tissue. Common disorders include, but are not limited to, phenylketonuria (1), renal insufficiency (2), epilepsy (3), osteoporosis (4), hyperpeptiduria (5) and the like.

For purposes of this application, "gastrointestinal disorders" are defined as any condition or manifestation that involves the gut, and the mastication of materials in the body including, but not limited to, the large intestine, small intestine, gall bladder, and liver. Common disorders are to include, but are not limited to, ulcers (7), polyps (8), pernicious anemia (9), Crohn's syndrome(10), diarrhea(11), acute bowel disorder (ABD) (12) and the like.

For purposes of this application, "central nervous system disorders" are defined as any condition or manifestation that involves the brain, spinal column, nerve or nerve tissue, or the coating of nerves, and the transfer or processing of information in the body or changes in behavior. Common examples are, but not limited to, schizophrenia(13), dementia(14), Parkinson's disease(15), multiple sclerosis(16), and the like. Central nervous system disorders, and Autism Spectral Disorders (ASD) in particular, are lifelong conditions affecting communication, learning and social skills. Autism Spectral Disorders include, but are not limited to, Attention Deficit Disorder (ADD), Attention Deficit Hyperactivity Disorder ADHD), Asperger's Syndrome, Pervasive Developmental Disorder (PDD) and autism, and the like.

For purposes of this application, "autoimmunity or reduced immunity disorders " are defined as any condition or manifestation that involves changes in one's immune system, specifically to mount a response against one's self. The immune response may be produced by any material, endogenous or foreign, and may follow vaccination or infection. Common disorders include, but are not limited to, celiac disease(18), diabetes(19), and HIV-related disorders(20) and the like. Although in most cases the victims of ASD are not afflicted with mental retardation, they do suffer from severe developmental disabilities.

For purposes of this application, cancer(21) is defined as "a popular generic term for malignant neoplasms, a great group of diseases of unknown and probably multiple causes, occurring in all human and animal populations and arising in all tissues composed of potentially dividing cells. The basic characteristic of cancer is the transmissible abnormality of cells that is manifested by reduced control over growth and function leading to serious adverse effects on the host through invasive growth and metastases.

Historically, autism spectral disorders have been a scientific enigma in regard to both treatment and diagnosis, largely due to the number of overlapping disorders that make up these syndromes.

Notable characteristics of individuals clinically diagnosed with an autism spectral disorder include: repetitive bodily motions, perseveration, extreme self-absorption, acute sensory differences, and severely limited diet, often to include products containing casein and gluten.

Early work suggested a relationship between aberrant behavior (schizophrenia, autism) and the ingestion of certain foods, particularly wheat and milk. In these and other studies many patients appeared improved, by the removal of these foods from their diet. Chromatography of urine obtained from children afflicted with an ASD revealed the presence of common peaks. These workers have proposed, without further identification, that these common peaks resulted from the presence of small, food-derived peptides. Because the proposed peptides have known opiate-like activity, they were theorized to be the cause of the patients' dysfunctional behavior.

Historically, these Disorders, including the autism spectral disorders, have been a scientific enigma in regard to both treatment and diagnosis, largely due to the number of overlapping disorders that make up these syndromes. Autism, for example, is a diagnosis based only on behaviors. These behaviors constitute the final neurological pathway of a number of syndromes that have been, for convenience, labeled autism or the autism spectral disorder.

Recently, specific syndromes have been culled out of the ASD, such as PKU, Landau-Kleffner syndrome, Fragile X syndrome, Rett's syndrome, and Purine Autism. Some are treatable through various means. Further, there is evidence that many forms of autism may be related to immune system dysfunction, as indicated by the appearance of Rubella Autism, CMV-related Autism, and HIV-related Autism.

Because the ASD have complex etiologies, it has been difficult to develop specific diagnostic measures. One form of ASD that potentially encompasses a large proportion of autistic patients has been related to gluten/casein intolerance. When placed on a gluten/casein-free diet, children evidently afflicted with this form of ASD have shown improvements ranging from mild to dramatic. There are scattered reports documenting the complete recovery of younger children (usually under two) who have been placed on a gluten/casein-free diet.

Biochemical methods for diagnosing many of these human disorders, including specific forms of ASD, have not been available. In particular, it would be useful to provide biochemical methods for the specific diagnosis of human disorders such as multiple sclerosis, Parkinson's disease, Alzheimer's dementia, and ASD relating to gluten/casein intolerance. It is important to identify children who may be afflicted with diet-related autism so that effective treatment may be initiated as early as possible.

### Summary of the Invention

The present invention broadly relates to human disorders including, but not limited to, metabolic and nutritional disorders, gastrointestinal disorders, central nervous system disorders, and autoimmunity reduced immunity disorders or cancer.

The present invention more specifically relates to diagnostic markers comprising isolated peptides originally found in human tissue and body fluids and derivatives thereof. These peptides have been found to consistently exhibit opiate-like activity and/or are derivatives of peptides that exhibit opiate-like activity.

The present invention also relates to methods for diagnosis of these disorders by identification of these peptides, as well as methods for detection of these peptides. Methods include, but are not limited to, Immunoassay methods and Mass Spectrometry methods.

In particular, the present invention relates to methods for diagnosing human disorders, such as a diet-related form of autism, by detection or quantitation of the associated peptides.

The present invention also relates to pharmaceutical compositions for treatment of human disorders.

Specifically, diagnosis of central nervous system disorders, such as autism spectral disorders, is usually made on the basis of behavioral analysis. A need exists for diagnosing these human disorders by detection and/or quantitation of substances specific to particular types of human disorders that are present in biological samples derived from persons so afflicted. As noted above, it is particularly useful to provide biochemical methods that enable the diagnosis and/or treatment of autism spectral diseases related to gluten/casein intolerance. This need has been met by the present invention. We have discovered that body tissues and body fluids, in particular urine samples from a subset of patients diagnosed with autism, or classified as having an autism spectral disorder, contain a variety of opiate-like and/or opiate derived peptides.

For purposes of this application, "autism spectral disorder" is defined as any disorder selected from the group related to autism, pervasive developmental disorders, Asperger's syndrome, Attention Deficit Disorder (ADD) and Attention Deficit Hyperactivity Disorder (ADHD) .

For purposes of this application, "diagnostic marker" is defined as a marker which exhibits the following characteristics:
a) is specific to a given population;
b) can be measurable and the measurement can be reproduced; and
c) is persistent for the time necessary to be detected in a biological sample.

For purposes of the application, "receptor" is defined as any of the various sensory nerve endings in, but not limited to, skin, deep tissues, viscera, or special sense organs.

For purposes of this application, "opiate-like peptide" is defined as any molecule, either proteinaceous or non-proteinaceous, which comprises an amino-terminal end and a carboxyl-terminal end that binds to an opiate or "opiate-like" receptor. The "opiate-like" peptide may be an agonist or antagonist. Examples of opiate-like peptides include, but are not limited to, morphine, casomorphin, dermorphin, naloxone, proenkephalin, enkephalins and endorphins.

For purposes of this application, "opiate-derived peptide" is defined as any peptide that is a derivative of an opiate-like peptide but which may not necessarily exhibit opiate and/or opiate-like activity.

For purposes of this application, "opiate-like" receptor is defined as any proteinaceous or non-proteinaceous material that may be cell surfaced or internal which binds "opiate-like" and/or opiate derived peptides, and which in turn frequently triggers a response either locally or remotely within the body. "Opiate-like receptors", by definition, would include opiate receptors. Examples of "opiate-like receptors" include, but are not limited to, mu (m), kappa (k), and delta (d) opiate receptors. These are coupled to G-proteins and may be homoleptic or chimeric. Further, there are subtypes of each receptor type. There is ~60% amino acid identity among the sequences of the m, d, and k opioid receptors. The sequences of the putative membrane spanning segments and the three intracellular loops connecting these segments are highly conserved, whereas the sequences of the extracellular NH2-terminal segments second and third loops and the intracellular COOH-terminal are divergent. It is reasonable to assume these divergent extracellular regions may be responsible for the distinct ligand binding behavior for the m, d, and k opioid receptors.

Mu (m) receptors have a high affinity for morphine and the antagonist naloxone. Delta (d) receptors mediate the biological effects of endogenous neurotransmitters, the enkephalins and the antagonist naltrindole. Kappa (k) receptors mediate the physiological actions of the endogenous transmitters, the dynorphins, and the antagonist nor-BNI. The different types of opioid receptors are expressed in distinct regions of the CNS and behavioral evidence indicates that they may mediate selective pharmacological actions of opiates and "opiate-like" peptides.

For purposes of this application, "agonist' is defined as a drug or binder capable of combining with receptors to initiate drug actions; it possesses affinity and intrinsic activity.

For purposes of this application "antagonist" is defined as a drug or binder capable of opposing the action of other materials. It neutralizes or impedes the actions or effects of agonists.

We have identified the peptides, as discussed below, some of which are known to be neurologically active. We have also determined the amino acid sequences of the peptides.

In a first embodiment, the present invention provides clinicians with diagnostic markers for a wide variety of potential disorders including, but not limited to: metabolic and nutritional disorders, gastrointestinal disorders, central nervous system disorders, autoimmunity or reduced immunity disorders, and cancer which are characterized by the presence or absence of certain peptides in body fluids such as urine.

In a second embodiment of the invention, the present invention relates to methods for diagnosing a wide variety of human disorders including, but not limited to: metabolic and nutritional disorders, gastrointestinal disorders, central nervous system disorders, autoimmunity or reduced immunity disorders, and cancer which are characterized by the presence or absence of certain peptides in body fluids such as urine.

In a third embodiment, the present invention relates to methods for detecting diagnostic markers comprising opiate-like peptides. The methods include, but are not limited to: immunoassay methods, ELISA assay methodology, kits, and test devices for detecting the presence and/or quantity of opiate-like peptides in biological samples.

For purposes of this application, "biological samples" are defined, but not limited to, epithelial fluids, tissue, serum, whole blood, feces, tears, cerebrospinal fluid and urine.

More specifically, the present invention relates to a high pressure liquid chromatography (HPLC) method for determining, in patient urine samples, the presence of peptides associated with a diet-related form of autism associated with gluten/casein intolerance.

The present invention also relates to a method of using mass spectrometry for unambiguously identifying these opiate-like peptides and characterizing biological representative samples. Biochemical indicators, specifically peptides exhibiting opiate-like activity, can be detected. Some of the peptides are known to be neurologically active and may be responsible for some of the observed behaviors associated with specific human disorders such as autism. Peptides that have been identified in a subset of urine samples obtained from patients characterized as autistic and are known to be neurologically active include: β-casomorphin (13), α-gliadin (14), dermorphin (15), deltorphin I, deltorphin II (16), and morphine modulating neuropeptide (17).

In a fourth embodiment, the present invention relates to methods of treating these human disorders such as for example autism.

In a fifth embodiment, the present invention relates to a pharmaceutical composition for treatment of these disorders.

In a sixth embodiment of the invention, the present invention relates to antagonists and agonists of "opiate-like" receptors and methods for screening for antagonists and agonists.

### Brief Description of the Figures:

Figure 1 shows a high pressure liquid chromatogram of a normal urine sample. Peaks designated by A, B and C comprise the reference profile for a normal urine sample.

Figure 2 shows a high pressure liquid chromatogram of an autistic urine sample. The presence of "opiate-like" and/or "opiate-derived" diagnostic marker peaks are shown in the range from 15-30 minutes, and the changes in the reference profile peaks, as compared to Figure 1, characterize the patient as autistic.

Figure 3 shows a high pressure liquid chromatogram of an autistic urine sample. The diagnostic marker peaks occur in the range from 15-30 minutes.

Figure 4 shows a high pressure liquid chromatogram of a clinically confirmed "recovered" autistic patient. The reference profile peaks are depicted, as well as the absence of the diagnostic marker peaks.

Figures 5-8 show electrospray mass spectrometric analysis of patient urine sample fractions, obtained from reversed-phase HPLC. Each spike represents a single peptide of a designated molecular weight, and the neuropeptide identified in each figure is labeled accordingly.

Figure 9 shows the identification of peptides eluting in the biomarker region, using the combination of reversed-phase HPLC fractionation and ES-MS analysis of the fractions.

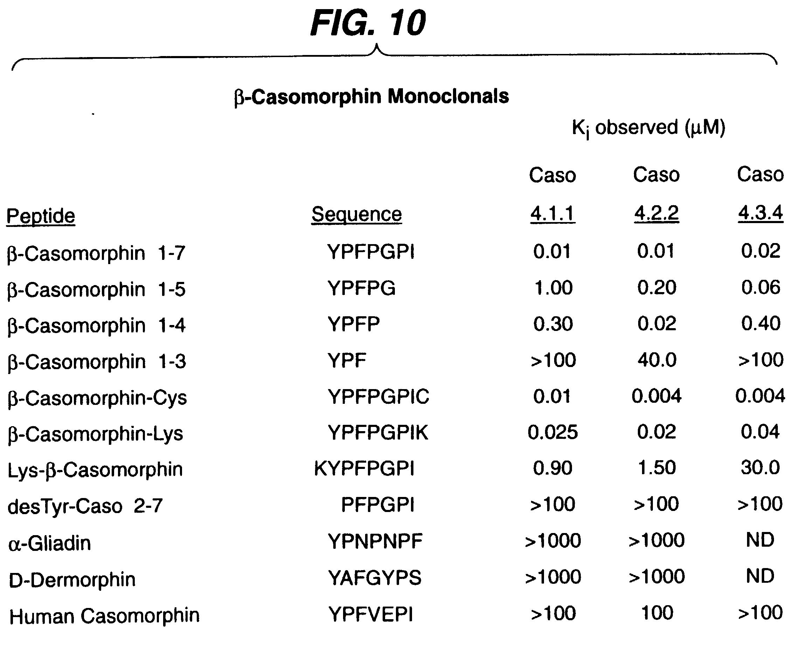

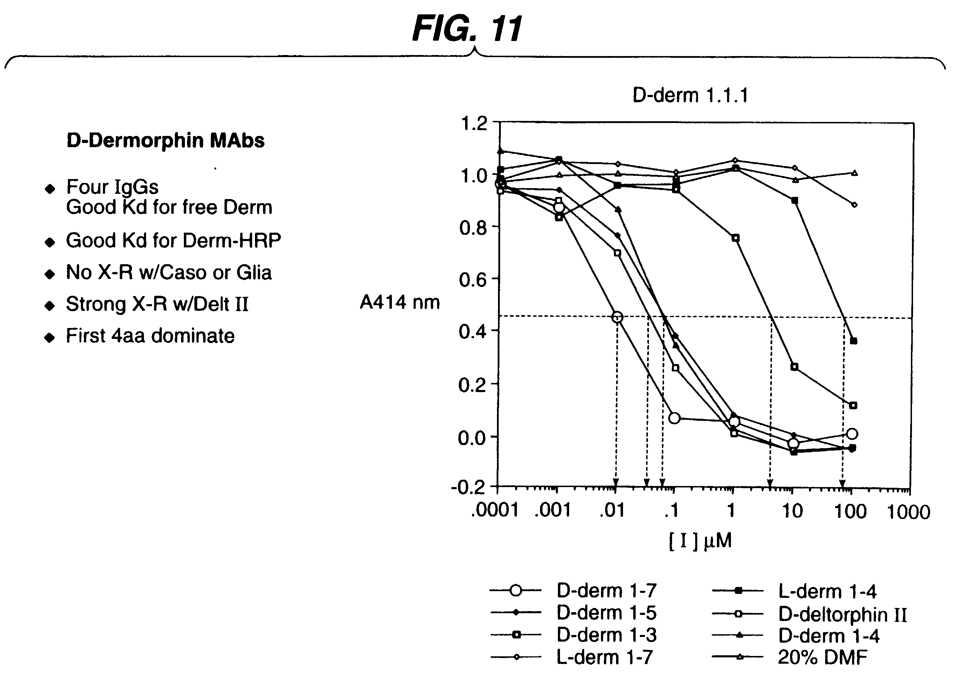

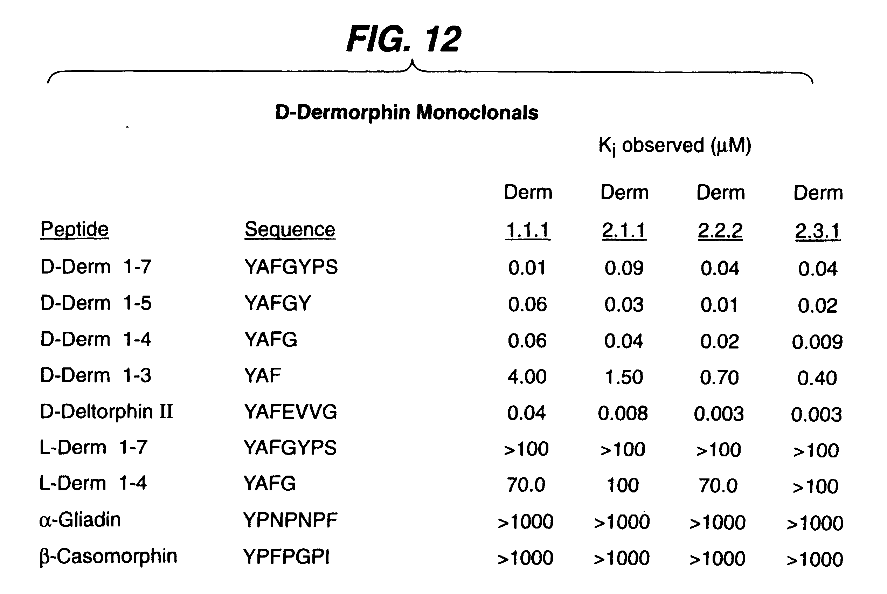

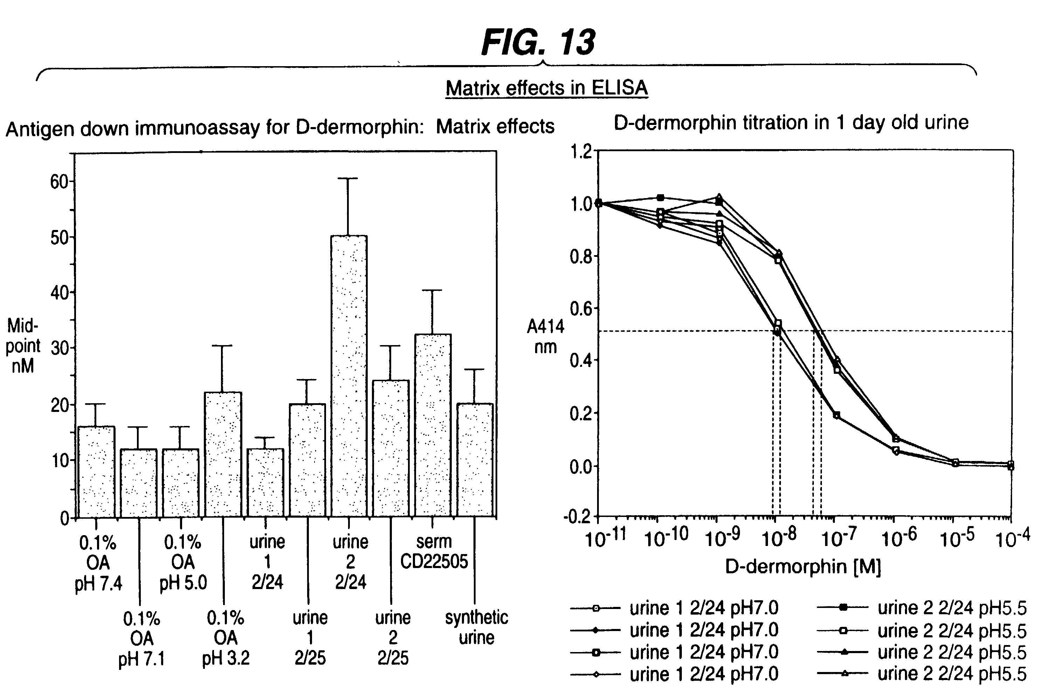

Figures 10-13 show ELISA data for monoclonal antibodies.

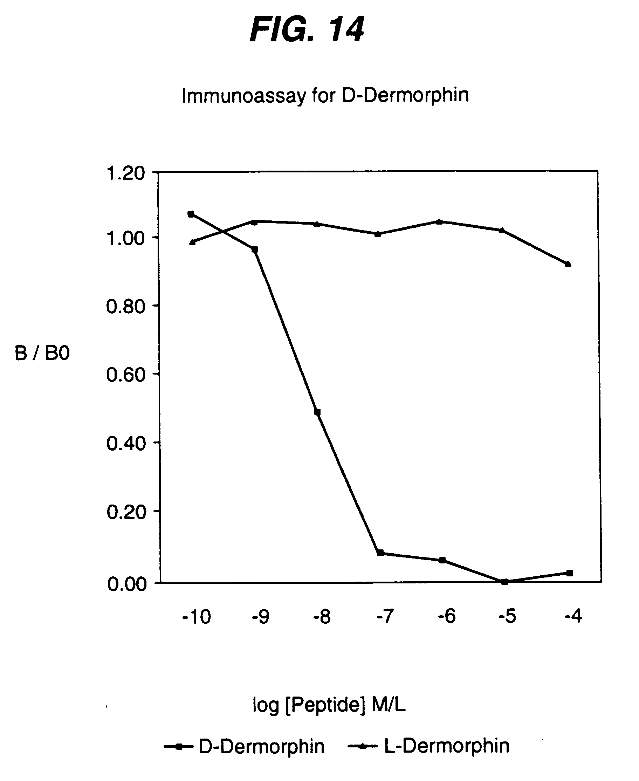

Figure 14 shows an Immunoassay for D-Dermorphin. Dose-response curves for D-dermorphin (squares) and L-dermorphin (triangles) were generated using the antigen-down immunoaasay described in the text. B/B₀ is the ratio of the measured A₄₁₄ at each peptide concentration divided by the measured A₄₁₄ when the concentration of peptide was zero.

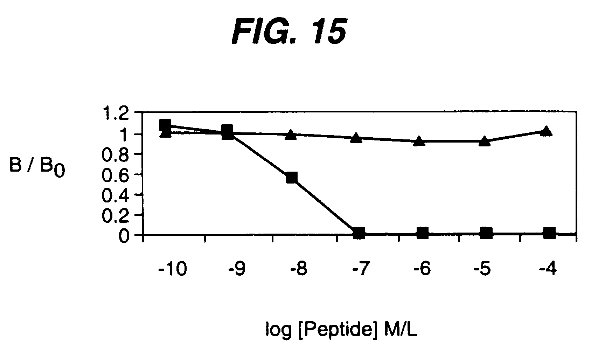

Figure 15 shows an Immunoassay for β-casomorphin. Dose-response curves for β-casomorphin (squares) and Tyr2caso, β-casomorphin with the first two amino acids reversed (triangles), were generated using the antigen-down immunoassay described in the text. B/B₀ is the ratio of the measured A₄₁₄ at each peptide concentration divided by the measured A₄₁₄ when the concentration of peptide was zero.

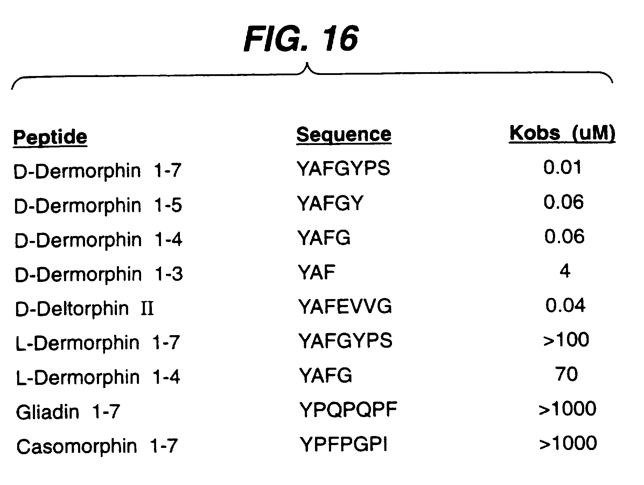

Figure 16 shows Cross-reactivity of Derm 1.1.1. The K_{obs} for each of a variety of peptides was determined by performing the antigen-down assay described in the text. K_{obs} is the concentration of peptide required to reduce the measured A₄₁₄ to 50% of that measured when the concentration of inhibiting peptide is zero. Derm 1.1.1 is highly specific for the amino-terminal four amino acids of D-dermorphin (all L-amino acids except for D-alanine in the second position), highly cross-reactive with deltorphin II, has little reactivity with L-dermorphin (all L-amino acids), and has essentially no reactivity with β-casomorphin or α-gliadin.

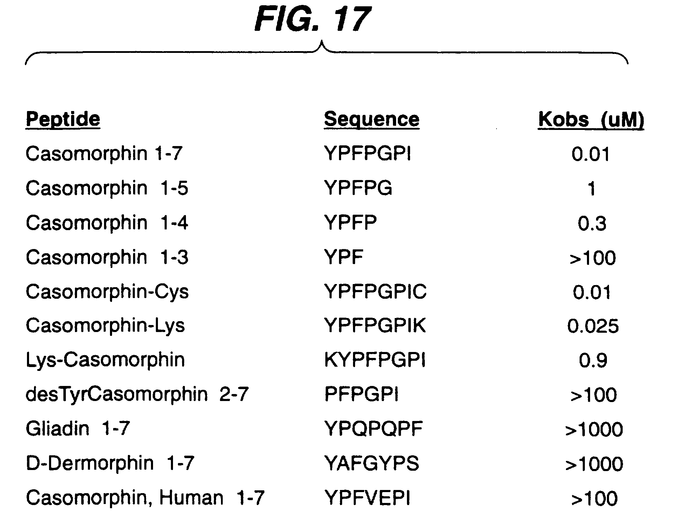

Figure 17 shows Cross-reactivity of Caso 4.2.2. The K_{obs} for each of a variety of peptides was determined by performing the antigen-down assay described in the text. K_{obs} is the concentration of peptide required to reduce the measured A₄₁₄ to 50% of that measured when the concentration of inhibiting peptide is zero. Caso 4.2.2 is highly specific for the amino-terminal four amino acids of bovine β-casomorphin, and has little cross-reactivity with α-gliadin, dermorphin or human casomorphin.

### Detailed Description of the Invention:

The following list of "opiate-like" peptides and/or "opiate-derived" peptides were identified in a subset of urine samples obtained from patients diagnosed as autistic. They are listed under the parent protein or parent peptide, which was determined as described below. The list is representative of "opiate-like peptides" and "opiate-derived peptides" and are not meant to be limiting in any way.

The peptides observed and listed under the parent protein or parent peptide, as well as the others listed therein, may, in some cases, result from bacterial and/or enzymatic degradation of the parent protein or peptide. Fresh urine samples may only present the parent peptide (or protein). Alternatively, the peptides may result from endogenous metabolism. At present, the origin of some of these peptides is not known. It should be noted that Hydroxyproline [HYP](3-Hydroxyproline and 4-Hydroxyproline) are represented in the sequence listing (CRF) as Xaa.

For purposes of this application "derivative" is intended to indicate a naturally occurring degradation product of the parent peptide, including endogenous and sample degradation. Alternatively, a derivative is intended to mean a polypeptide which is derived from the parent peptide by suitably modifying the DNA sequence coding region for the parent peptide to yield a variant. The derivative may also occur by the addition of one or more amino acids at either or both the C- and N- terminal ends of the native amino acid sequence, substitution of one or more amino acids at one or more sites in the native amino acid sequence, deletion of one or more amino acids at either or both ends of the native sequence or at one or more sites within the native sequence, or insertion of one or more amino acids in the native sequence, including all side chain deletions or conformational changes. The derivative, thus, could be an "opiate-like peptide" or "opiate-derived peptide".

### Casein (parent protein)

β-Casomorphin (parent peptide)
(NH4-TyrProPheProGlyProIle-COOH) (SEQ. ID. NO. 1)
(NH4-TyrProPheProGlyPro-COOH) (SEQ. ID. NO. 2)
(NH4-TyrProPheProGly-COOH) (SEQ. ID. NO. 3)
(NH4-TyrProPhePro-COOH) (SEQ. ID. NO. 4)

(NH4-TyrProPhe-COOH)                    (SEQ. ID. NO. 5)
(NH4-ProPheProGlyProIle-COOH)           (SEQ. ID. NO. 6)

And derivatives thereof.

Gluten (parent protein)
α-Gliadin (parent peptide)
(NH4-TyrProGlnProGlnProPhePro-COOH) (SEQ. ID. NO. 7)
(NH4-TyrProGlnProGlnPro-COOH) (SEQ. ID. NO. 8)
(NH4-TyrProGlnProGln-COOH) (SEQ. ID. NO. 9)
(NH4-TyrProGlnPro-COOH) (SEQ. ID. NO. 10)
(NH4-TyrProGln-COOH) (SEQ. ID. NO. 11)
(NH4-ProGlnProGlnProPhe-COOH) (SEQ. ID. NO. 12)

And derivatives thereof.

Dermorphin (parent peptide)
(NH4-TyrAlaPheGlyTyrProSer-COOH) (SEQ. ID. NO. 13)
(NH4-TyrAlaPheGlyTyrPro-COOH) (SEQ. ID. NO. 14)
(NH4-TyrAlaPheGlyTyr-COOH) (SEQ. ID. NO. 15)
(NH4-TyrAlaPheGly-COOH) (SEQ. ID. NO. 16)
(NH4-TyrAlaPhe-COOH) (SEQ. ID. NO. 17)
(NH4-AlaPheGlyTyrProSer-COOH) (SEQ. ID. NO. 18)

And derivatives thereof.

Deltorphin II (parent peptide)
(NH4-TyrAlaPheGluValValGly-COOH) (SEQ. ID. NO. 19)
(NH4-TyrAlaPheGluValVal-COOH) (SEQ. ID. NO. 20)
(NH4-TyrAlaPheGluVal-COOH) (SEQ. ID. NO. 21)
(NH4-TyrAlaPheGlu-COOH) (SEQ. ID. NO. 22)
(NH4-AlaPheGluValValGly-COOH) (SEQ. ID. NO. 23)

And derivatives thereof.

Morphine modulating neuropeptide (parent peptide)
(NH4-PheLeuPheGlnProGlnArgPhe-COOH (SEQ. ID. NO. 24)
(NH4-PheLeuPheGlnProGlnArg-COOH) (SEQ. ID. NO. 25)
(NH4-PheLeuPheGlnProGln-COOH) (SEQ. ID. NO. 26)
(NH4-PheLeuPheGlnPro-COOH) (SEQ. ID. NO. 27)
(NH4-PheLeuPheGln-COOH) (SEQ. ID. NO. 28)
(NH4-PheLeuPhe-COOH) (SEQ. ID. NO. 29)

And derivatives thereof. 
Deltorphin I (parent peptide)
(NH4-TyrAlaPheAspValValGly-COOH) (SEQ. ID. NO. 30)

Novel Autism Peptides I
(NH4-TyrAlaPheAspValVal-COOH) (SEQ. ID. NO. 31)
(NH4-TyrAlaPheAspVal-COOH) (SEQ. ID. NO. 32)
(NH4-TyrAlaPheAsp-COOH) (SEQ. ID. NO. 33)
(NH4-AlaPheAspValValGly-COOH) (SEQ. ID. NO. 34)
(NH4-AlaProPheHypGlyHypLle-COOH) (SEQ. ID. NO. 35)
(NH4-AlaProPheHypGlyHyp-COOH) (SEQ. ID. NO. 36)
(NH4-AlaProPheHypGly-COOH) (SEQ. ID. NO. 37)
(NH4-AlaProPheHyp-COOH) (SEQ. ID. NO. 38)
(NH4-AlaProPhe-COOH) (SEQ. ID. NO. 39)
 
(NH4-AlaPro-COOH)                    (SEQ. ID. NO. 40)
(NH4-ProPheHypGlyHypLle-COOH)        (SEQ. ID. NO. 41)

Novel Autism Compounds II
(NH4-TyrProPheProProLle-COOH)        (SEQ. ID. NO. 42)
(NH4-TyrGlyGlyTyr-COOH)              (SEQ. ID. NO. 43)
 The following chart pertains to the above peptide sequences and their actual analysis. Please note that what is listed on this chart is the M+1 mass values.

| **Amino acid sequence** | **MW** | **Fragment profile** | **Mass Only** |
|---|---|---|---|
| **Beta-Casomorphin:** | | | |
| Tyr-Pro-Phe-Pro-Gly-Pro-Ile | (789) | + | |
| Tyr-Pro-Phe-Pro-Gly-Pro | (676) | | + |
| Tyr-Pro-Phe-Pro-Gly | (579) | Not found | |
| Tyr-Pro-Phe-Pro | (522) | | + |
| Tyr-Pro-Phe | (425) | Not found | |
| Pro-Phe-Pro-Gly-Pro-Ile | (626) | | + |
| | | | |

| **Alpha-Gliadin :** | | | |
|---|---|---|---|
| Tyr-Pro-Gln-Pro-Gln-Pro-Phe -Pro | (949) | | + |
| Tyr-Pro-Gln-Pro-Gln-Pro-Phe | (875) | + | |
| Tyr-Pro-Gln-Pro-Gln-Pro | (728) | | + |
| Tyr-Pro-Gln-Pro-Gln | (631) | | + |
| Tyr-Pro-Gln-Pro | (503) | | + |
| Tyr-Pro-Gln | (406) | | + |
| Pro-Gln-Pro-Gln-Pro-Phe | (694) | | + |
| Tyr-Ala-Phe-Gly-Tyr-Pro-Ser | (803) | | + |
| Tyr-Ala-Phe-Gly-Tyr-Pro | (716) | Not found | |
| Tyr-Ala-Phe-Gly-Tyr | (619) | | + |
| Tyr-Ala-Phe-Gly | (456) | + | |
| Tyr-Ala-Phe | (399) | + | |
| Tyr-Ala-Phe-Ala-Tyr* | (633) | + | |
| Ala-Phe-Gly-Tyr-Pro-Ser | (640) | | + |

| **Deltorphin II** | | | |
|---|---|---|---|
| Tyr-Ala-Phe-Glu-Val-Val-Gly | (783) | | + |
| Tyr-Ala-Phe-Glu-Val-Val | (774) | | + |
| Tyr-Ala-Phe-Glu-Val | (675) | Not found | |
| Tyr-Ala-Phe-Glu | (546) | Not found | |
| Ala-Phe-Glu-Val-Val-Gly | (620) | | + |

| **Morphine Modulating Peptide** | | | |
|---|---|---|---|
| Phe-Lue-Phe-Gln-Pro-Gln-Arg-Phe | (1081) | | + |
| Phe-Lue-Phe-Gln-Pro-Gln-Arg | (934) | Not found | |
| Phe-Lue-Phe-Gln-Pro-Gln | (778) | | + |
| Phe-Lue-Phe-Gln-Pro | (650) | | + |
| Phe-Lue-Phe-Gln | (553) | | + |
| Phe-Lue-Phe | (425) | Not found | |

| **Deltorphin I** | | | |
|---|---|---|---|
| Tyr-Ala-Phe-Asp-Val-Val-Gly | (769) | | + |
| Tyr-Ala-Phe-Asp-Val-Val | (712) | Not found | |
| Tyr-Ala-Phe-Asp-Val | (613) | | + |
| Tyr-Ala-Phe-Asp | (512) | | + |
| Ala-Phe-Asp-Val-Val-Gly | (606) | Not Found | |

| | | | |
|---|---|---|---|
| * This compound is commonly referred to as [D-Ala] dermorphin fragment. | | | |

The following molecular weights (molecular ion ± 1) pertain to compounds that were discovered during the course of this study. The mass spec fragmentation patterns have been established and found to be consistent in many autistic samples. However, the identity of these compounds has not yet been determined.
733 MW
755 MW
511 MW
488 MW

463 MW
458 MW
441 MW
479 MW
Although the masses listed above have the mass of the corresponding peptide, it must be understood that other compounds may share this mass value.

The present embodiments of the invention would be understood by those skilled in the art to include either or both chiral forms. Both D-Ala or L-Ala forms may be present in equal or in essentially equal amounts, or only one of the D or L isomeric forms may have been present or predominate the mixture. This aspect is unimportant, however, in their detection using methods that do not distinguish on the basis of chiral differences.

The abundance of these opiate-like and/or opiate-derived peptides in the urine of autistic children suggests that these children share a deficiency in an enzyme that would normally be involved in the catabolism of small peptides. Elaborating on this idea, it is probable that the deficient enzyme is unique in its activity (no other enzyme has similar or overlapping substrate specificity), the product of a single gene (there are not multiple isoenzymes) and is primarily involved in catabolic events not essential for survival under normal conditions.

As an ensemble, the peptides described above exhibit several distinctive traits. Specifically, most are 6-8 amino acids long, many have N-terminal tyrosines and penultimate prolines, and some contain multiple alternating prolines. The prominence of prolines suggests that an enzyme involved in the digestion of small proline-rich peptides may be deficient in some ASD children.

Dipeptidyl peptidase IV/(DPPIV)CD26 has all of these predicted traits and some other properties that suggest that it may be a primary deficiency. DPPIV/CD26 is generally an integral cell surface enzyme expressed on tissues involved in the uptake of small peptides including intestinal brush border, the bile canicular membrane domains of the liver and the cortex of the kidney. It specifically cleaves the N-terminal dipeptide from many peptides containing penultimate prolines (Yaron and Naider, 1993). In fact, one of its characteristic substrates is β-casomorphin (Kreil et al, 1983; Heymann and Mentlein, 1986).

Several other properties of DPPIV/CD26 are consistent with its potential critical role in autistic children. It is unique in its activity among enzymes that are cell-surface associated, it is encoded by a single gene located on chromosome 2 (Mathew et al, 1994), and rats with a homozygous genetic deficiency in DPPIV/CD26 exhibit no overt pathology (Erickson et al, 1992; Tirrupathi etal, 1993). Another remarkable property of DPPIV/CD26 is its expression on T-lymphocytes, including CD4-positive T helper cells, the population targeted by HIV. Some HIV-infected patients develop a conspicuous HIV-associated autism (Moss et al, 1994; Brouwers et al, 1995). Further, DPPIV/CD26 is also the cell surface binding protein for adenosine deaminase (De Meester et al, 1994; Kameoka et al 1993), another enzyme associated with autism.

Deficiencies in DPPIV/CD26 can arise by a variety of mechanisms. The first is a primary genetic deficiency where the encoded enzyme is, for some reason, not expressed or dysfunctional. The second is that an autoimmune response could include antibodies that inhibit the function of the otherwise normal enzyme. Finally, it is conceivable that an infectious agent, possibly even a one of the gut flora, could produce a compound that inhibits the activity of DPPIV/CD26.

If any of these mechanisms are operating in autistic children, it is perfectly reasonable to rescue the children from the effects of the presumably causal gut deficiency by oral supplementation of mammalian or non-mammalian DPPIV, in much the same way as lactase is administered to those with lactose intolerance. Thus, the present invention is directed to a pharmaceutical composition comprising DPPIV/CD26.

The present invention is further illustrated in the following examples, which is not intended in any way to limit the scope of the invention as claimed.

### EXAMPLE 1

### Isolation of "Opiate-Like" Peptides and "Opiate-Derived" Peptides

### Biological Sample Collection

Biological samples, represented by urine, were collected from children clinically diagnosed with Attention Deficit Disorder (ADD), Attention Deficit Hyperactivity Disorder (ADHD), Asperger's Syndrome, Pervasive Developmental Disorder (PDD), or autism, as well as from "normal"¹ children to be used as reference samples in determining differences in urine composition that are specifically related to ASD.
¹ Since children develop at different rates, typical children were chosen from a group randomly, who had no developmental or social delays and had no unusual dietary regimen.

Into a sterile polypropylene specimen container, 0.5 g thymol (Sigma T-0501) was added as a preservative, and approximately 40 mL of urine was collected from each patient. Samples were frozen or processed immediately.

### Solid Phase Extraction Method:

Samples that had been frozen were thawed in the refrigerator or at room temperature, but never in a 37°C water bath, which was proven to cause sample degradation in the time of thawing. Each sample, thawed or fresh, was spun at 1000 rpm in a tabletop centrifuge for five minutes, then filtered with a 0.22 µm syringe filter and a 10 mL syringe, with a sample volume of greater than 10 mL.

The Sep-Pak cartridge was prepared with multiple solvent flushes. Two 10 mL aliquots of methanol were applied first, in a drop-like fashion, allowing time for the solvent to equilibrate in the cartridge, cleaning it. The methanol was then rinsed out with two 10mL flushes of water, also dropwise. One 10 mL aliquot of 0.1% TFA, aqueous, was then applied slowly to equilibrate the column into the necessary conditions to enhance peptide binding during sample loading. Air was blown onto the cartridge to remove excess fluid between each successive flush, with the exception of the final flush of 0.1% TFA, after which several drop of solvent were left in the syringe to prevent any air from reaching the column. The sample was applied to the cartridge immediately, with the cartridge remaining wet from the previous 0.1% TFA flush to maximize peptide interactions with the cartridge matrix.

Ten mL of the filtered urine sample was loaded dropwise onto the prepared cartridge, with the flow going to waste, contained and disposed of as biohazardous waste. Two 5mL aliquots of 0.1% TFA, aqueous, were applied to flush the cartridge of any unbound material, with air flushes between each solvent flush to dry the matrix. Approximately 8mL of 0.15% CH₃CN+0.1% TFA, aqueous, was then dripped slowly through the cartridge, allowing time for the CH₃CN to interact with the matrix and remove loosely bound materials. If the cartridge eluate was still tinted with color after the 8mL volume has been applied, more solvent was added in 1 mL increments until the eluate was clear, indicating all peptides have been eluted that would desorb at that level of hydrophobicity. The cartridge was then blown dry completely, until no bubbles were formed by the air. The final peptide elution occurred with two 5mL flushes of 0.1% TFA in 100% CH₃CN, and the eluate was collected. This flush was done very slowly, with the solvent allowed to equilibrate to completely desorb the peptides (the cartridge remained in the solvent for several minutes without flushing). The cartridge was blown dry at the end of the final flush to insure that all the peptides have been removed.

The final eluate was placed in a 40°C water bath under nitrogen gas and allowed to evaporate to dryness. The processed sample was then reconstituted with 500 µL of 15% CH₃CN+0.1% TFA, aqueous, and agitated vigorously with a vortex mixer to completely dissolve the peptide sample. A 100 µL aliquot was removed for reversed-phase HPLC analysis.

### High Pressure Liquid Chromatography Experimental Conditions:

Reversed phase chromatography on a Waters Symmetry C18 column was used to analyze the urine samples for peptides that could be used as biochemical markers for ASD syndromes. Solvent A consisted of 0.1% TFA aqueous (V/V) and solvent B was 0.1% TFA in 95% acetonitrile/5% water. The elution gradient was as follows:

| Time (min.) | %A | %B |
|---|---|---|
| 0 | 100 | 0 |
| 8 | 95 | 5 |
| 40 | 50 | 50 |
| 60 | 0 | 100 |
| 70 | 95 | 5 |
| 80 | 100 | 0 |
| 90 | 100 | 0 |

Flow rate for the gradient was 1.4 mL/min., sample injection volume was 20 µL, and UV detection was set at 215 nm.

### HPLC Experimental Results

Five bioactive peptides were used as standards and their retention times were determined: β-casomorphin (Sigma C-5900), oxytocin (Sigma 0-4375), glucagon (Sigma G-3157), leucine enkephalin (Sigma L-9133), and somatostatin (Sigma S-9129). Having established reproducible retention times for these peptides, the urine samples were analyzed, and peaks eluting at the same time as the standard peptides were considered for further analysis. As the HPLC method was specifically designed for the detection of peptides, the substances in the urine samples having retention times similar to the standards were assumed to be peptides.

HPLC does not provide definitive proof of identity. However, electrospray mass spectrometry (ES-MS) can definitively establish chemical identity. The substances, presumed to be peptides, eluting in the region of the standard peptides, were definitively identified as peptides and their amino acid sequence were determined using ES-MS, as described below. Once the amino acid sequences of the peptides were established, we endeavored to determine their origin, that is, the parent molecule(s) from which they were evidently derived, as discussed below.

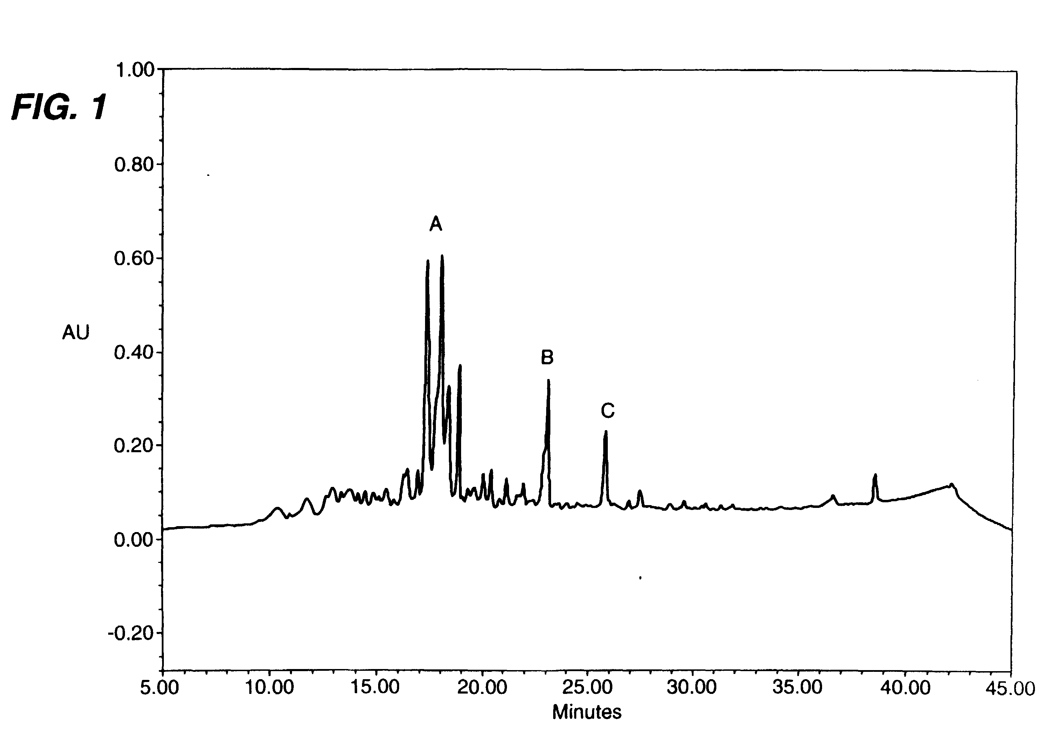

A series of peaks exhibiting several characteristic profiles appeared in the samples from the normals and all the patients diagnosed as autistic (referred to hereinafter as normal reference peaks or profiles). The largest reference profile was a quadruplet of peaks having retention times from 16-19 minutes (Figure 1A). This grouping of peaks was seen in every sample, with slight deviations in the relative heights of the peaks. A second single reference peak was observed having an elution time of 23 ± 1 minutes (Figure 1B), and its height also varied among samples. A third peak was observed, with an elution time of 25 ± 1 minutes, and with a height also varying relative to the quadruplet region (Figure 1C). The combination of all these peaks formed the reference profile.

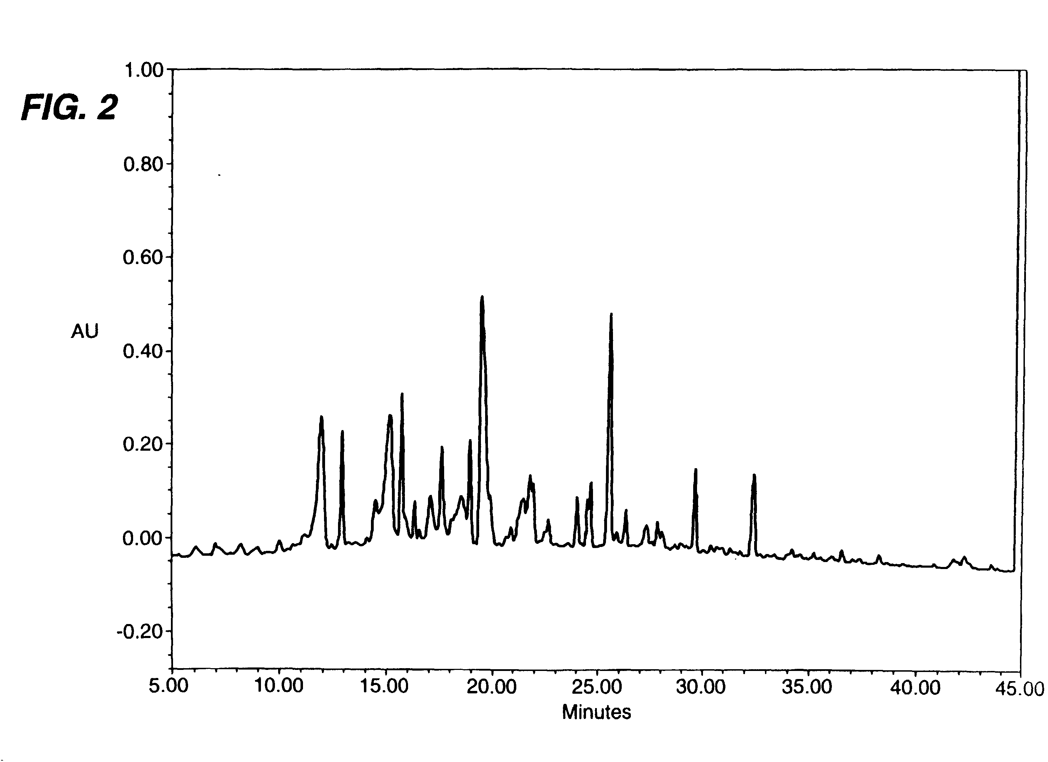

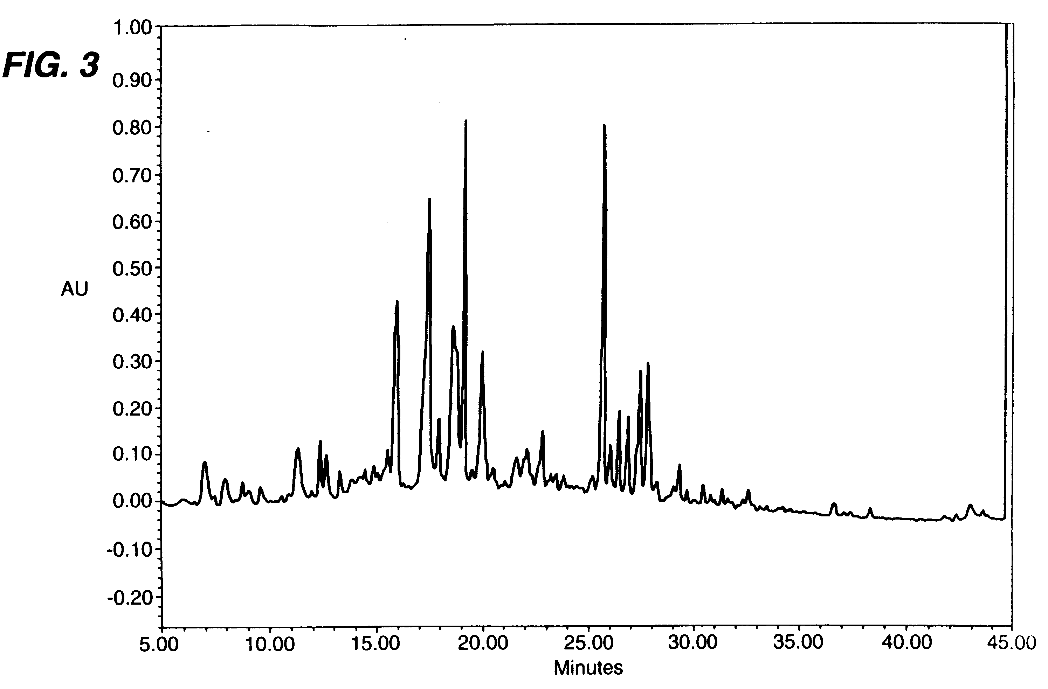

The urine samples were classified into three groups based on their source and chromatographic behavior. The first group observed were the normal samples, which contained the reference profile of peaks. The second group observed was a subset of samples from patients with a diagnosed ASD. The chromatograms from this group were indistinguishable from the normals. A third group, composed of samples from patients with a diagnosed ASD, exhibited biomarker peaks which eluted between 15-30 minutes, in addition to the normal reference peaks. The biomarker peaks eluted in the same region as the standard peptides noted above (Figures 2 and 3).

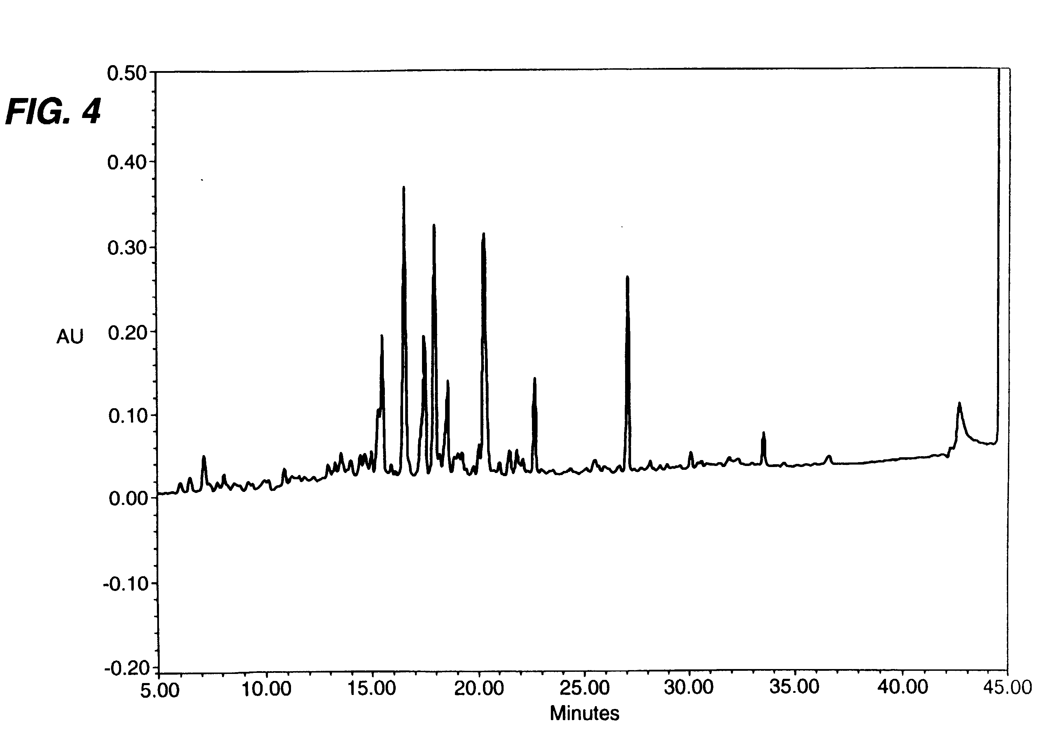

One urine sample, from a child diagnosed with autism who was subsequently placed on a gluten and dairy-free diet, showed an absence of the biomarker peaks. This sample did contain the reference peak profiles observed with the normal urines (Figure 4). The absence of biomarker peaks in this sample, if correlated with the presence or absence of ASD symptomology, would lead one to the conclusion that the patient should no longer exhibit the behavioral symptoms associated with ASD. In fact, this child has been clinically declassified from ASD, and is considered cured of autism.

All the peptides associated with the biomarker peaks in the third group of urine samples have not been linked to bioactivity. However, their presence in urine of patients diagnosed with an ASD and absence in normal samples enables the HPLC method described above to be used as a means for identifying these peptides as biomarkers for what is ostensibly a diet-related form of ASD associated with gluten/casein intolerance. The HPLC/ES-MS combined method (discussed below) provides a means to specifically identify these peptides (based on their linear amino acid sequences), and families thereof, as being biochemically correlated with this form of autism. It is not known if the presence of the peptides deltorphin II, dermorphin and morphine modulating neuropeptide (and their derivatives) is diet-related; however, they were always found in association with the casein and gluten related species. Thus, their presence in urine is diagnostic of the gluten/casein intolerance form of autism.

It was extremely surprising to find dermorphin, deltorphin II, and morphine modulating neuropeptide (and derivatives thereof)in the patient samples. These peptides were present along with the casein/gluten family and diagnostic for the gluten/casein intolerance form of ASD, as stated. However, their presence may be related to other neurological disorders as well, such as Multiple Sclerosis (MS). They may also be related to HIV infection and/or associated with gut stress. We observed that these peptides are more stable in urine than the gluten/casein related peptides (although this was not studied this in a systematic manner). Therefore, these peptides are considered particularly useful biomarkers for diagnosing the casein/gluten intolerance form of ASD.

### Electrospray Mass Spectrometry Experimental Results

ES-MS was used to confirm the peptide nature and determine the amino acid sequences of the species represented by the biomarker peaks observed using HPLC.

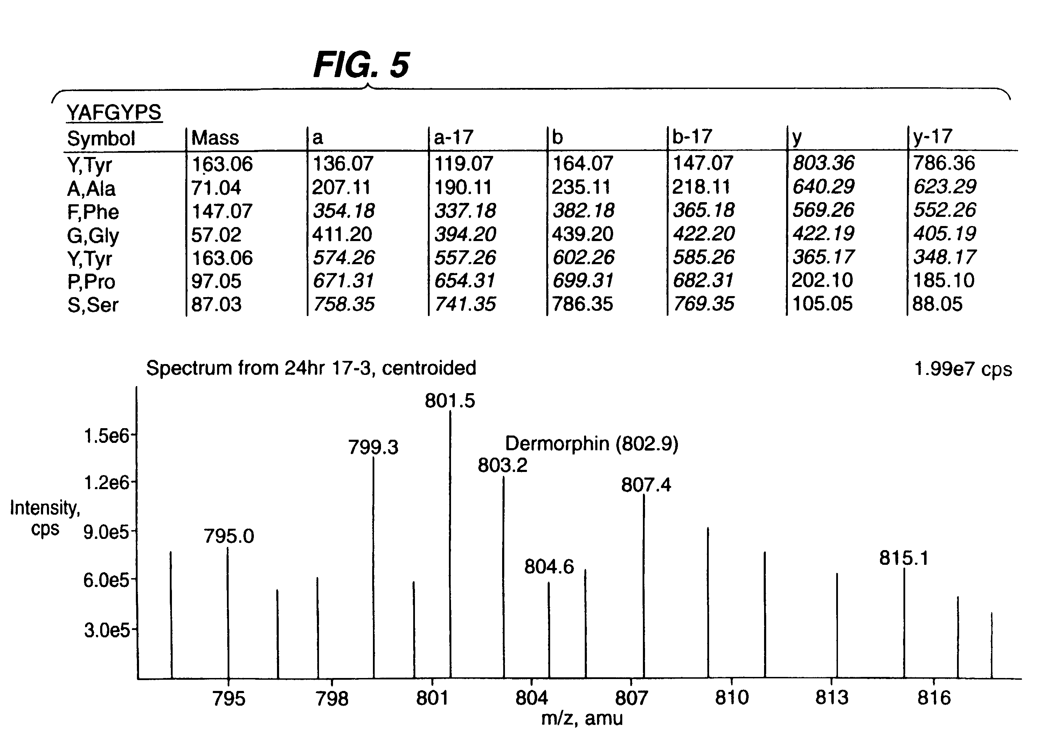

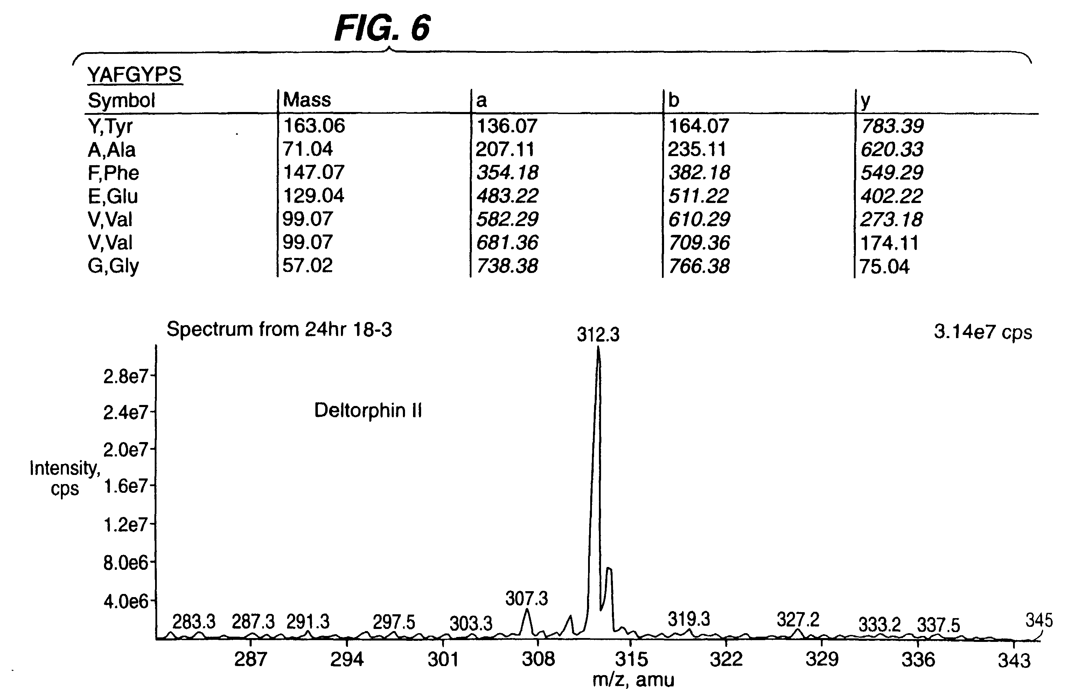

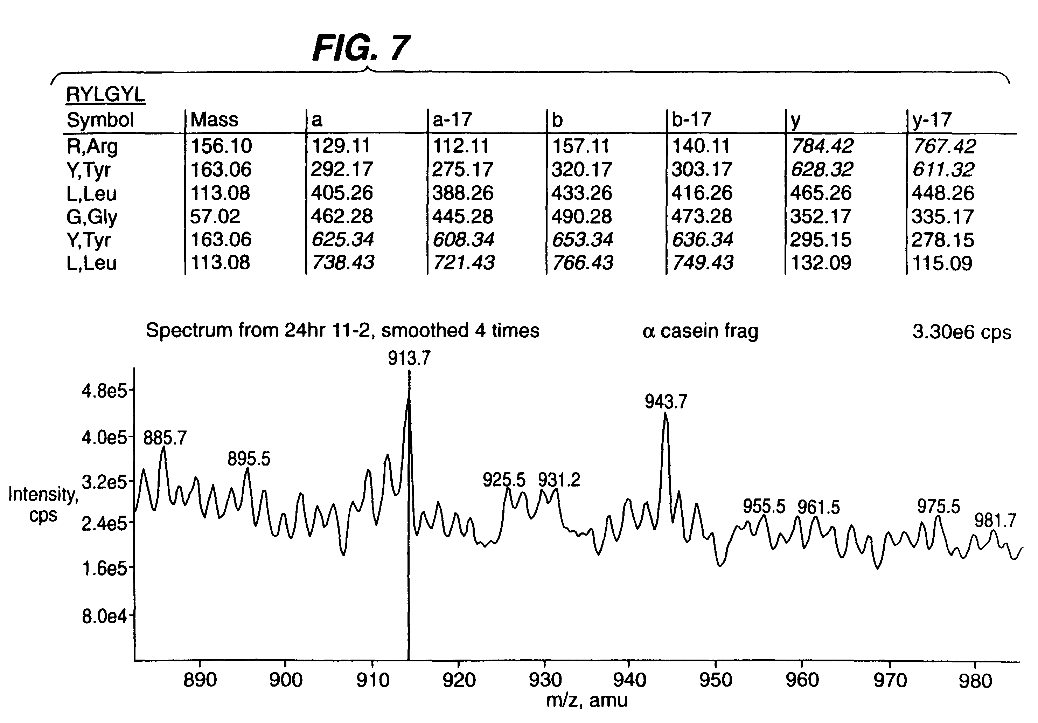

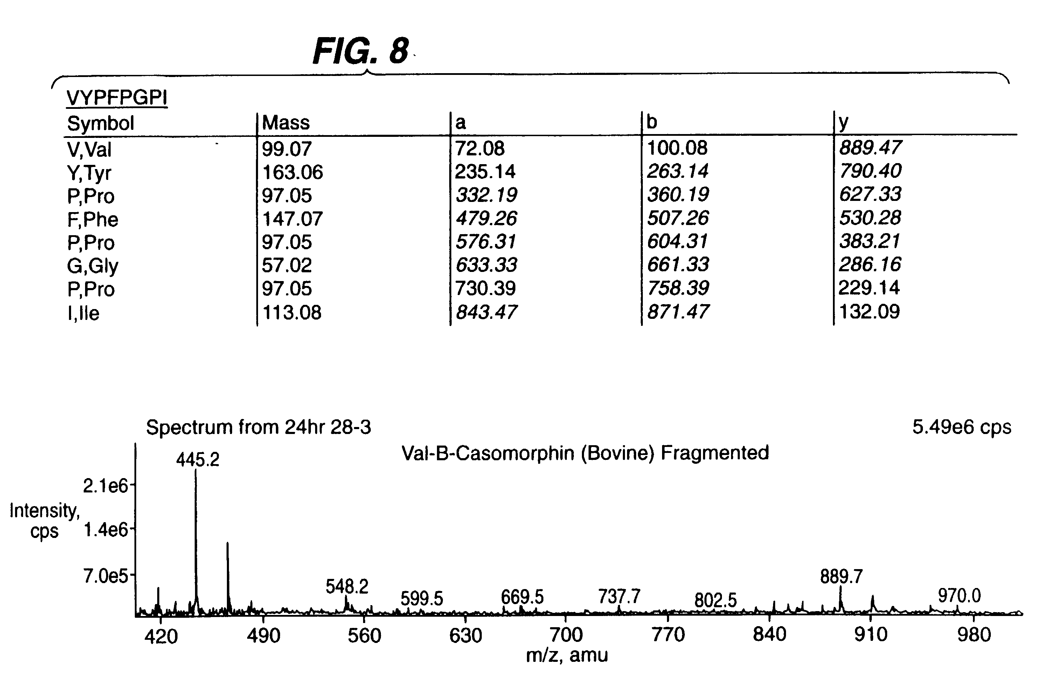

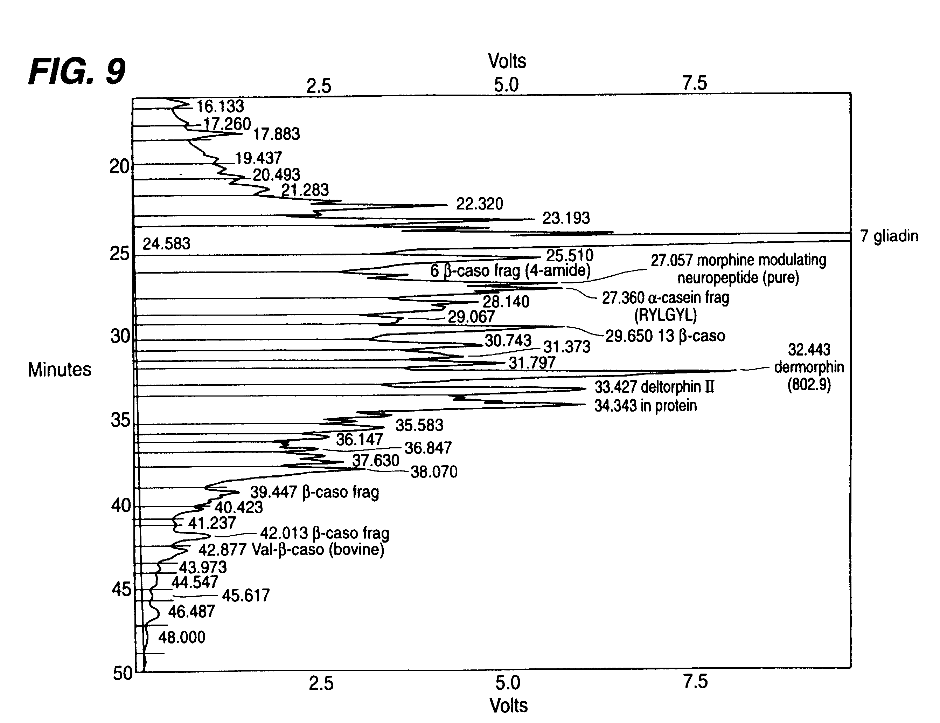

ES-MS provided both the exact molecular masses of the peptides and their amino acid sequences. Preparative-scale reversed phase chromatography (under the HPLC conditions described above), was used to obtain specific fractions having retention times within the biomarker region, which were then analyzed using ES-MS (Figures 5-8). Once the chemical identities of the peptides were established, the amino acid sequences of the peptides were compared with amino acids sequences of proteins available in protein data bases to determine potential parent proteins, and thus potential origins of the peptides. A search engine called "Peptide Scan," provided by Perkin Elmer/Sciex, was employed for the comparative analysis, utilizing the European Molecular Biological Labs (EMBL) Non-Redundant Database (NRDB). Combinations of peptide fragments eluting in the bioactive region occur simultaneously in the samples and are easily identified in this manner (Figure 9).

Using ES-MS, we established that the normal urine samples (the first group) and the second group of urine samples did not contain any peptides in the biomarker elution region, while the samples from the third group contained multiple peptides in that region. Peptide fragments of the food proteins gluten and casein (wheat and milk proteins, respectively), as well as peptides of unknown origin, such as deltorphin II, dermorphin, and morphine modulating neuropeptide, were present in the samples from the third group. The peptide fragments of the food proteins were abundant, but the presence of dermorphin, deltorphin II and morphine modulating neuropepide in all the third group samples was startling, as mentioned. None of the normal samples or samples in the second group contained any of the food protein fragments, nor did they contain any of the unexpected peptides as disclosed in the present application.

In summary, using HPLC and electrospray mass spectrometry, we have separated and identified many high concentration peptides from the urine of 25 ASD patients. In a large percentage of the ASD samples (13 of the 25) we found high concentrations of low molecular mass, opiate-like peptides, notably α-gliadin from wheat gluten (NH4-TyrProGlnProGlnProPhe-COOH) and β-casomorphin from bovine casein (NH4-TyrProPheProGlyProIle-COOH). In addition, further breakdown products of α-gliadin and β-casomorphin, including their N-terminal 4- and 5-mers, as well as their des-Tyr forms, were conspicuously abundant. We also found other opiate-like peptides that were not clearly derived from food sources, including dermorphin (NH4-TyrAlaPheGlyTyrProSer-COOH), which has 1000 times the opiate activity of morphine, deltorphin II (NH4-Tyr AlaPheGluValValGly-COOH) and morphine-modulating neuropeptide (NH4-PheLeuPheGlnProGlnArgPhe-COOH).

The discovery of the aforementioned peptides in the urine of ASD patients provides a biochemical means to confirm diagnosis of a dietary-induced form of ASD ostensibly related to gluten/casein intolerance. Our discovery enables clinicians to readily diagnose this particular diet-related ASD using any appropriate biochemical method. The peptides identified thus far have known opiate-like activity and can stimulate behaviors similar to those induced by morphine and other opiate agonists. The stimulation of morphine receptors could create the unusual behaviors associated with ASD.

With the identification of biomarker peptides in patient urine, it could be possible to predict dietary influences on behavior. Elimination of certain foods could aid the patient in the recovery from developmental disorders. The presence of other peptides, with nonspecific or undefined origins, could indicate additional developmental disorders, as well as potential treatments.

### EXAMPLE 2

### Development of Immunoassays for Marker Peptides

*Synthesis of peptides.* β-casomorphin and D-dermorphin were synthesized on an automated peptide synthesizer by conventional methods. Each was prepared in two forms: 1) the native peptide as a model antigen and calibrator (hereafter, Caso and Derm), and 2) the native peptide with an additional cysteine residue on the carboxyl terminus (hereafter, Caso-Cys and Derm-Cys). The purity and sequence of each synthetic peptide was determined by electrospray mass spectrometry.

*Conjugation of peptides* to *protein carriers.* For the purposes of immunization, screening and immunoassay building, the Caso-Cys and Derm-Cys peptides were conjugated to keyhole limpet hemocyanin (KLH), bovine serum albumin (BSA) and horseradish peroxidase (HRP), respectively. Briefly, each protein was reacted with a heterobifunctional crosslinker which had a maleimide group on one end and an N-hydroxysuccinimide ester on the other (50-fold molar excess, 0.1 M KPO₄, pH7.5, 2 hours, RT). After exhaustive dialysis against 0.1 M KPO₄, pH7.0, with 5 mM EDTA, the activated protein was reacted with a 50-fold molar excess of Caso-cys or Derm-cys (0.1 M KPO₄, pH7.0,with 5 mM EDTA, RT, 20 hours). Excess free peptide was removed by size exclusion chromatography on Sephadex G-25. The extent of conjugation was assessed by molecular weight shifts in SDS-PAGE.

*Immunization of mice*. 100 µg of either Caso-cys-KLH or Derm-cys-KLH in Ribi adjuvant was administered by intraperitoneal injection into six-to-twelve week old female CAF1 mice at three-week intervals. After three injections a final intraperitoneal injection of 100 µg immunogen in phosphate-buffered saline (PBS) was administered three to five days before sacrifice for hybridoma making. One week after each administration of antigen, each mouse was bled and its serum was screened for antipeptide antibodies by the ELISA test described below.

*Generation and screening of hybridomas.* Selected animals were terminated and their spleens were removed aseptically. Splenocytes were dispersed, red blood cells removed by hypotonic lysis, and the resulting leukocytes were washed extensively with Dulbecco's Modified Eagle's Medium before fusion with SP2/0-Ag14 cells essentially by the method of Lane (Lane RD 1985. J Immunol Meth 81, 223-228). The resulting hybridomas were distributed into 96-well microculture plates and the resulting cultures were screened after 10-14 days growth in HAT-medium.

*Selection of monoclonal antibodies.* Each microculture well was screened for the presence of antiCaso or anti-Derm antibodies by conventional enzyme linked immunosorbant immunoassays (ELISA). Wells of 96-well microassay plates were coated with 100 µL of PBS containing either Caso-cys-BSA or Derm-cys-BSA (2 µg/mL) and then blocked with 1% ovalbumin in PBS. After washing with wash buffer (PBS with 0.05% Tween 20), the plates were either stored frozen at -20 °C or used for screening. 50 µL from each microculture was transferred to a corresponding well in a coated microassay plate and allowed to incubate for 45-60 minutes at RT with continuous shaking. The plates were then washed 5 times with wash buffer and 50 µL containing HRP-conjugated goat antimouse heavy and light chain antibody (HRP-GAM) was added to each well. After incubation for 45-60 min, the plates were washed 5 times and 50 µL ABTS substrate was added to each well. HRP activity representing the presence of antiCaso or antiDerm antibodies was detected by monitoring absorbance at 414 nm on a microplate reader. Wells with absorbances greater than two times background were selected for further examination.

*Reactivity with soluble free peptide.* The ability of each antibody to react with the free peptide as well as its conjugated form was assessed by simple competition in the ELISA format described above. Selected hybriboma culture supernates were prepared at three dilutions: neat, 1:5 and 1:50 by dilution with PBS containing 0.1% ovalbumin (PBS-OA). Each sample was then mixed with an equal volume of PBS-OA containing either the Caso or Derm peptides at 2 mM, 2 µM or 2 nM. The resulting mixtures were assayed on Caso-BSA or Derm-BSA coated ELISA plates exactly as described above. Those supernates that had high absorbances at the greatest dilution and were completely competed by the soluble peptides were selected for further characterization.

*Isotype analysis of selected Derm and Caso antibodies.* The heavy and light chain isotype of each antibody was determined using the same ELISA format described above except that each sample was incubated in eight separate wells and then each well was developed with a different HRP conjugate including HRP-GAM, antimouse µ, γ₁, γ₂ₐ, γ_{2b}, γ₃, and α heavy chains and κ and λ light chains. Those wells with any of the γ heavy chains were selected for further analysis.

*Affinity analysis of antipeptide antibodies.* To determine the approximate affinity of each Mab for its cognate peptide, each selected culture supernate was first titrated in the ELISA format described above to ensure that each Mab would be tested at a concentration sufficiently dilute that no saturation effects would mask the competition between the immobilized peptide conjugate and the free soluble peptide. As described above, appropriately diluted selected culture supernates were mixed with equal volumes of peptides in PBS-OA at concentrations between 2 mM and 2nM. The concentration of free peptide that yielded 50% reduction in absorbance at 414 nm was considered the apparent affinity for the free peptide, or k_{obs}. Antibodies with k_{obs} < 1 µM were selected for further analysis.

*Cross-reactivity analysis.* To determine the specificity of each mAb, the affinity test described above was repeated substituting a variety of other peptides for Caso or Derm. For casomorphin immunoassays, crossreactivity (k_{obs} < 0.1 mM) with Derm, α-gliadin or deltorphin II were unacceptable. For dermorphin immunoassays, crossreactivity (k_{obs} < 0.1 mM) with Caso, and α-gliadin were unacceptable, but significant cross-reactivity with Deltorphin II was considered acceptable because of the similar structure of the two molecules.

*Cloning and stabilization* of *selected hybridoma cell lines.* Cells from four wells containing antiDerm secreting hybridomas and three containing antiCaso secreting hybridomas were cloned in soft agarose. Individual colonies were transferred to 96-well microculture plates, allowed to grow for two-to-five days and then rescreened by ELISA for secretion of antiDerm or antiCaso antibodies. Three clones from each cell line were frozen for long term storage in liquid nitrogen.

*Design of immunoassays.* Two types of immunoassays were constructed for the quantitation of Derm and Caso in biological fluids. The first, referred to as the Antigen Down Format, is identical to the ELISA described above. Peptide-BSA conjugates were adsorbed onto the wells of 96-well microtiter plates. Plates were the blocked with PBS-1%OA, washed and stored frozen. Antipeptide antibody, either impure in the unprocessed culture supernate or purified by affinity chromatography on Protein A-Sepharose, was titrated as described above to ensure that no saturation effects would obscure competition between the immobilized peptide-BSA and the free peptide in the sample. Standard samples were prepared by making dilutions of peptide in PBS-OA at concentrations ranging from 2 mM to 2 nM. Equal volumes of appropriately diluted antipeptide were mixed with the peptide standards, and 50 µL of each mixture was transferred to one of the prepared peptide-BSA coated plates. After incubation (RT on a shaker) for 45-60 minutes, the plates were washed 5 times with wash buffer. 50 µL of HRP-GAM was added to each well, incubated a further 45-60 minutes, washed and then the plate was developed by adding 50 µL ABTS substrate to each well and monitoring color development at 414 nm on a standard microplate reader. The resulting standard curve indicated that concentrations below 10 nM could be easily detected and that concentrations above 1 µM completely prevented the antipeptide antibody from binding to the peptide-BSA conjugate adsorbed on the plate. The antigen down format is a simple, robust format that can detect either Caso or Derm at concentrations between 1 nM and 1 µM.

The second format is called the RaMFc format. Microtiter plates were coated with Rabbit antimouse IgG Fc polyclonal antibody (RaMFc) and then the plates were blocked with PBS-1%OA, washed and stored frozen. Thawed plates were washed and then each well was incubated with 50 µL of antipeptide mAb (either impure in the unprocessed culture supernate or purified by affinity chromatography on Protein A-Sepharose). After about 30-60 minutes in which the antipeptide mAb was bound by the immobilized RaMFc, the plates were washed. Samples were mixed with peptide-HRP (1 nM) and transferred to the prepared microtiter wells. After a 30-60 minute incubation (RT on a shaker), the plates were washed. The assay was developed by the addition of 50 µL of ABTS substrate and quantitated by monitoring absorbance at 414 nm on a standard microplate reader. The RaMFc format produced an immunoassay that detected peptides at about 1 nM and was completely inhibited between 0.1 and 1 µM.

### References Cited in Application:

All references cited in this application are herein incorporated by reference.
1. Correlation between cerebrospinal fluid phenylalanine and beta-endorphin in patients with phenylketonuria. Bach FW, Nielsen JB, Buchholt J, Lou H, Güttler F Neurosci Lett 1991 Aug 5 129:1 131-3
2.
   a) Endothelin antagonists in salt-dependent hypertension associated with renal insufficiency. Doucet J, Gonzalez W, Michel JB J Cardiovasc Pharmacol 1996 May 27:5 643-5.
   b) George E. Brown memorial lecture. Role of atrial peptides in body fluid homeostasis. Ballermann BJ, Brenner BM Circ Res 1986 May 58:5 619-30
3.
   a) Mu-opiate receptors measured by positron emission tomography are increased in temporal lobe epilepsy. Frost JJ, Mayberg HS, Fisher RS, Douglass KH, Dannals RF, Links JM, Wilson AA, Ravert HT, Rosenbaum AE, Snyder SH, et al Ann Neurol 1988 Mar 23:3 231-7
   b) Neuropeptides and seizures. Snead OC 3d Neurol Clin 1986 Nov 4:4 863-75 c) Endogenous opioid peptides and epilepsy. Ramabadran K, Bansinath M Int J Clin Pharmacol Ther Toxicol 1990 Feb 28:2 47-62.
4.
   a) Aging, neuroendocrine function, and osteoporosis. Peterlik M Exp Gerontol 1997 Jul-Oct 32(4-5):577-86
   b) Amylin, calcitonin gene-related peptide, calcitonin, and adrenomedullin: a peptide superfamily. Wimalawansa SJ Crit Rev Neurobiol 1997 11:2-3 167-239
   c) Urinary N-telopeptide levels discriminate normal, osteopenic, and osteoporotic bone mineral density [see comments] Schneider DL, Barrett-Connor EL Arch Intern Med 1997 Jun 9 157:11 1241-5
   d) Analysis of immunoreactive and biologically active human parathyroid hormone-peptides by high-performance-liquid-chromatography. Schettler T, Aufm, Kolk B, Atkinson MJ, Radeke H, Enters C, Hesch RD Acta Endocrinol (Copenh) 1984 Sep 107:1 60-9
5. Nature and consequences of hyperpeptiduria and bovine casomorphins found in autistic syndromes. Reichelt K.L., Knivsberg, A.M., Nodland, M., Lind, G. Dev Brain Dysfunct 1994 7:71-85.
6. Endogenous opiates: 1996. Olson GA, Olson RD, Kastin AJ Peptides 1997 18:10 1651-88
7.
   a) The effect of centrally administered neuropeptides on the development of stress-induced gastric ulcers in rats. Hernandez DE, Nemeroff CB, Orlando RC, Prange AJ Jr J Neurosci Res 1983 9:2 145-57.
   b) Protective action of endogenous opioid-like peptides of different origins in duodenal ulcer in rats Vinogradov VA, Polonski VM Biull Eksp Biol Med 1985 May 99:5 548-9.
   c) Oral famotidine: a potential treatment for children with autism. Linday, L.A. Medical Hypotheses 1997 48:381-386.
8. Hyperplastic polyps: a cell lineage which both synthesizes and secretes trefoil-peptides and has phenotypic similarity with the ulcer-associated cell lineage. Hanby AM, Poulsom R, Singh S, Jankowski J, Hopwood D, Elia G, Rogers L, Patel K, Wright NA Am J Pathol 1993 Mar 142:3 663-8
9.
   a) Amino-acids and peptides in human gastric juice with particular reference to pernicious anaemia: a review. Heathcote JG, Washington R Nature 1965 Aug 28 207: 941-4.
   b) Neuroendocrine tumors of the gastrointestinal tract. Chejfec G, Falkmer S, Askensten U, Grimelius L, Gould VE Pathol Res Pract 1988 Apr 183:2 143-54.
10.
   a) Receptors for sensory neuropeptides in human inflammatory diseases: implications for the effector role of sensory neurons. Mantyh PW, Catton MD, Boehmer CG, Welton ML, Passaro EP Jr, Maggio JE, Vigna SR Peptides 1989 May-Jun 10:3 627-45.
   b) Trefoil peptides: a newly recognized family of epithelial mucin-associated molecules. Poulsom R, Wright NA Am J Physiol 1993 Aug 265:2 Pt 1 G205-13
   c) Production of peptides inducing chemotaxis and lysosomal enzyme release in human neutrophils by intestinal bacteria in vitro and in vivo. Chadwick VS, Mellor DM, Myers DB, Selden AC, Keshavarzian A, Broom MF, Hobson CH Scand J Gastroenterol 1988 Jan 23:1 121-8
   d) Trefoil peptide gene expression in small intestinal Crohn's disease and dietary adaptation. Poulsom R, Chinery R, Sarraf C, Van Noorden S, Stamp GW, Lalani EN, Elia G, Wright NA J Clin Gastroenterol 1993 17 Suppl 1 S78-91
11. Diagnosing diarrhea in adults: a practical approach. McCray, W.H., Krevsky, B Hospital Medicine 1998 34(4):27-28, 29-30, 32, 35-36.
12.
   a) Activin A: a novel player and inflammatory marker in inflammatory bowel disease? Hübner G, Brauchle M, Gregor M, Werner S Lab Invest 1997 Oct 77:4 311-8
   b) Trefoil peptide gene expression in gastrointestinal epithelial cells in inflammatory bowel disease. Wright, NA, Poulsom, R, Stamp, G, Van Noorden, S, Sarraf, C, Elia, G, Ahnen, D, Jeffrey, R, Jeffrey, Longcroft, J, and Pike, C. Gatroenterology 1993 104: 12-20.
   c) Colonic vasoactive intestinal peptide nerves in inflammatory bowel disease. Kubota Y, Petras RE, Ottaway CA, Tubbs RR, Farmer RG and Fiocchi C. Gastroenterology 1992 102:1242-1251.
   d) Intestinal vessels express a high density of somatostatin receptors in human inflammatory bowel disease. Reubi JC, Mazzucchelli L, and Laissue JA Gastrenterology 1994 106:951-959.
13.
   a) Opioid Receptor Antagonists in Psychiatry: Beyond Drug Addiction. Jean-Philippe Rénéric and Manuel P. Bouvard CNS Drugs 1998 10: 365-382
   b) Some observations on the opiate peptides and schizophrenia. Watson SJ, Akil H, Berger PA, Barchas JD. Arch Gen Psychiatry 1979 Jan 36:1 35-41
   c) Dopamine Receptors and Dopamine Transporter in Brain Function and Addictive Behaviors: Insights from Targeted Mouse Mutants. John Drago, Poolpol Padungchaichot, Domenico Accili, Sara Fuchs Developmental Neuroscience 1998, 20:2-3:188-203.
   d) An open trial of risperidone in young autistic children. Nicholson B, Awad G, Sloman L. J. Am. Acad. Adolesc. Psychiatry 1198 April 37; 4:372-376.
14.
   a) Peptides and neurotransmission in the central nervous system. Tuominen M, Leppäluoto J Med Biol 1987 65:2-3 137-42
   b) Comparison of DSIP- (delta sleep-inducing peptide) and P-DSIP-like (phosphorylated) immunoreactivity in cerebrospinal fluid of patients with senile dementia of Alzheimer type, multi-infarct syndrome, communicating hydrocephalus and Parkinson's disease. Ernst A, Cramer H, Strubel D, Kuntzmann F, Schoenenberger GA J Neurol 1987 Oct 235:1 16-21
   c) Neuropeptides in cerebrospinal fluid in normal-pressure hydrocephalus and dementia. Wikkelsö C, Ekman R, Westergren I, Johansson B Eur Neurol 1991 31:2 88-93
   d) A correlation study of CSF neuropeptides in Alzheimer's and Parkinson's disease. Jolkkonen J, Hartikainen P, Soikkeli R, Bissette G, Nemeroff C, Riekkinen P Neuropeptides 1991 Jun 19:2 97-102
   e) Parkinson's disease and dementia: clinical and neurochemical correlations. Strittmatter MM, Cramer H Neuroreport 1992 May 3:5 413-6
15.
   a) A correlation study of CSF neuropeptides in Alzheimer's and Parkinson's disease. Jolkkonen J, Hartikainen P, Soikkeli R, Bissette G, Nemeroff C, Riekkinen P Neuropeptides 1991 Jun 19:2 97-102
   b) Primate model of Parkinson's disease: alterations in multiple opioid systems in the basal ganglia. Zamir N, Skofitsch G, Bannon MJ, Helke CJ, Kopin IJ, Jacobowitz DM Brain Res 1984 Nov 26 322:2 356-60
   c) Peptides in Parkinson's disease. Barbeau A Adv Exp Med Biol 1978 113: 101-10
   d) Restoration of ACTH/cortisol and LH responses to naloxone by chronic dopaminergic treatment in Parkinson's disease. Volpi R, Caffarra P, Scaglioni A, Maestri D, Chiodera P, Coiro V J Neural Transm Park Dis Dement Sect 1994 7:1 1-11
16.
   a) Do microbes with peptides mimicking myelin cause multiple sclerosis if the T cell response to their unique peptides is limited? Kaufman M J Theor Biol 1998 Aug 21 193:4 691-708
   b) Treatment and management of multiple sclerosis. Liversedge LA Br Med Bull 1977 Jan 33:1 78-83
   c) Correlation between milk and dairy product consumption and multiple sclerosis prevalence: a worldwide study. Malosse D, Perron H, Sasco A, Seigneurin JM Neuroepidemiology 1992 11:4-6 304-12
17.
   a) A reappraisal of the role of the various opiod receptor subtypes in cell-mediated immunity. Caroloeo MC, Arbitrio M, Melchiorri D, and Nistico G Neuroimmunomodulation 1994 1:141-147.
   b) Urinary levels of neopterin and biopterin in autism. Messahel S, Pheasant AE, Pall H, Ahmed-Choudhury J, Sungum-Paliwal RS and Vostanis P. Neuroscience Letters 1998 241:17-20.
   c) Brief report: dysregulated immune system in children with autism: beneficial effects of intravenous immunoglobulin on autistic characteristics. Gupta S, Aggarwal S, Heads C. Journal of Autism and Developmental Disorders 1996 26:439-452.
18.
   a) Jejunal proabsorptive actions of selective opiate agonists administered via the cerebral ventricles. Quito FL, Brown DR Neuropeptides 1989 Jul 14:1 39-44
   b) Effect of two synthetic alpha-gliadin peptides on lymphocytes in celiac disease: identification of a novel class of opioid receptors. Graf L, Horvath K, Walcz E, Berzetei I, Burnier J Neuropeptides 1987 Feb-Mar 9:2 113-22
19.
   a) Effects of beta-endorphin, met-enkephalin, and dynorphin A on basal and stimulated insulin secretion in the mouse. Ahrén B Int J Pancreatol 1989 Sep 5:2 165-78
   b) Central opiate modulation of growth hormone and insulin-like growth factor-I. Hashiguchi Y, Molina PE, Fan J, Lang CH, Abumrad NN Brain Res Bull 1996 40:2 99-104
20.
   a) Delta-opioid suppression of human immunodeficiency virus-1 expression in T cells (Jurkat). Sharp BM, Gekker G, Li MD, Chao CC, Peterson PK Biochem Pharmacol 1998 Aug 1 56:3 289-92
   b) Upregulation of HIV-1 expression in co-cultures of chronically infected promonocytes and human brain cells by dynorphin. Chao CC, Gekker G, Hu S, Sheng WS, Portoghese PS, Peterson PK Biochem Pharmacol 1995 Aug 25 50:5 715-22
   c) Host factors and the pathogenesis of HIV-induced disease. Fauci AS. Nature 1996 December 12 384:529-534.
21.
   a) Antiproliferative and receptor binding properties of α- and β-casomorphins in the T47D human breast cancer cell line. Hatzoglou A, Bakogeorgou E, Hatzoglou C, Martin P and Castanas E. European Journal of Pharmacology 1996 310:217-223.
   b) Opioid alkaloids and casomorphin peptides decrease the proliferation of prostatic cancer cell lines (LNCaP, PC3 and DU145) through a partial interaction with opioid receptors. Kampa M, Bakogeorgou E, Hatzoglou A, Damianaki A, Martin P, and Castanas E. European Journal of Pharmacology 1997 335:255-265.

## Claims

1. A diagnostic marker for a human disorder, comprising a peptide selected from the group consisting of:
(a) opiate-like peptide;
(b) opiate-derived peptide.

2. The diagnostic marker of claim 1 wherein the human disorder is an autism spectral disorder, such as autism, pervasive developmental disorder, Asperger's syndrome, Attention Deficient Disorder (ADD) or Attention Deficient Hyperactivity Disorder (ADHD).

3. The diagnostic marker of claim 1 wherein the human disorder is Multiple Sclerosis, Parkinson's disease or Alzheimer's dementia.

4. A diagnostic marker comprising at least one peptide sequence selected from the group consisting of:
SEQ ID NO: 1;
SEQ ID NO: 2;
SEQ ID NO: 3;
SEQ ID NO: 4;
SEQ ID NO: 5;
SEQ ID NO: 6; and
sequences having at least 30%, preferably at least 60% and more preferably at least 90% homology with at least one of the amino acid sequences from the above group.

5. A diagnostic marker comprising at least one peptide sequence selected from the group consisting of:
SEQ ID NO: 7;
SEQ ID NO: 8;
SEQ ID NO: 9;
SEQ ID NO: 10;
SEQ ID NO: 11;
SEQ ID NO: 12; and
sequences having at least 30%, preferably at least 60% and more preferably at least 90% homology with at least one of the amino acid sequences from the above group.

6. A diagnostic marker comprising at least one peptide sequence selected from the group consisting of:
SEQ ID NO: 13;
SEQ ID NO: 14;
SEQ ID NO: 15;
SEQ ID NO: 16;
SEQ ID NO: 17;
SEQ ID NO: 18; and
sequences wherein the peptide is at least 30%, preferably at least 60% and more preferably at least 90% homologous with at least one of the amino acid sequences from the above group.

7. A diagnostic marker comprising at least one peptide sequence selected from the group consisting of:
SEQ ID NO: 19;
SEQ ID NO: 20;
SEQ ID NO: 21;
SEQ ID NO: 22;
SEQ ID NO: 23; and
sequences having at least 30%, preferably at least 60% and more preferably at least 90% homology with at least one of the amino acid sequences from the above group.

8. A diagnostic marker comprising at least one peptide sequence selected from the group consisting of:
SEQ ID NO: 24;
SEQ ID NO: 25;
SEQ ID NO: 26;
SEQ ID NO: 27;
SEQ ID NO: 28;
SEQ ID NO: 29; and
sequences having at least 30%, preferably at least 60% and more preferably at least 90% homology with at least one of the amino acid sequences from the above group.

9. A diagnostic marker comprising at least one peptide sequence having the amino acid sequence of:
SEQ ID NO: 31;
SEQ ID NO: 32;
SEQ ID NO: 33;
SEQ ID NO: 34;
SEQ ID NO: 35;
SEQ ID NO: 36;
SEQ ID NO: 37;
SEQ ID NO: 38;
SEQ ID NO: 39;
SEQ ID NO: 40;
SEQ ID NO: 41;
SEQ ID NO: 42; and
SEQ ID NO: 43.

10. An ex-vivo method for diagnosing a human disorder, comprising identifying peptide, for instance as defined in any one of claims 1 to 9, having opiate-like activity in body tissue or body fluid.
